# EUROPEAN PATENT SPECIFICATION

(11) **EP 3 503 849 B1**
(45) Date of publication and mention of the grant of the patent: **08.03.2023**
(21) Application number: 17877041.8
(22) Date of filing: 04.12.2017
(51) Int. Cl.: A61F 2/24

(54) **LOW PROFILE HEART VALVE AND DELIVERY SYSTEM**
HERZKLAPPE MIT NIEDRIGEM PROFIL UND EINFÜHRUNGSSYSTEM
VALVULE CARDIAQUE À PROFIL BAS ET SYSTÈME DE POSE

(30) Priority: 02.12.2016 US 201662429680 P
(43) Date of publication of application: 03.07.2019
(73) Proprietor: Sinomed Cardiovita Technology Inc., 215104 Suzhou, Jiangsu Province (CN)
(72) Inventor: LI, Tianzhu, Tianjin 300457 (CN); MA, Jianxiang, Tianjin 300457 (CN); MENG, Lei, Tianjin 300457 (CN)
(74) Representative: Regimbeau
(86) International application number: PCT/CN2017/114381
(87) International publication number: WO 2018/099484

(56) References cited:
- EP-A1- 3 037 064
- WO-A1-2013/059747
- CN-A- 101 951 857
- CN-A- 103 108 611
- CN-A- 105 050 542
- CN-A- 105 476 731
- CN-A- 105 496 606
- CN-A- 105 496 608
- US-A1- 2011 137 397
- US-A1- 2014 350 666

## Description

### FIELD OF THE INVENTION

This invention generally relates to medical devices for replacing a patient's heart valve endovascularly or transcatheterly.

### BACKGROUND OF THE INVENTION

The human heart has four chambers and four valves. The heart valves control the direction of blood flow. Fully functional heart valves ensure that proper blood circulation is maintained during cardiac cycle. Heart valve regurgitation, or leakage, occurs when the leaflets of the heart valve fail to come fully into contact (coapt) due to disease, such as congenital, torn chordae tendineae, lengthened chordae tendineae, enlarged left ventricle, damaged papillary muscles, damaged valve structures by infections, degenerative processes, calcification of the leaflets, stretching of the annulus, increased distance between the papillary muscles, etc. Regardless of the cause, the regurgitation interferes with heart function since it allows blood to flow back through the valve in the wrong direction. Depending on the degree of regurgitation, this backflow can become a self-destructive influence on not only function, but also cardiac geometry. Alternatively, abnormal cardiac geometry can also be a cause of regurgitation, and the two processes may "cooperate" to accelerate abnormal cardiac function. The direct consequence of the heart regurgitation is the reduction of forward cardiac output. Depending on the severity of the leakage, the effectiveness of the heart to pump adequate blood flow into other parts of the body can be compromised.

Currently, the standard heart valve regurgitation treatment options include surgical repair/treatment and endovascular clipping. The standard surgical repair or replacement procedure requires open-heart surgery, use of cardio-pulmonary bypass, and stoppage of the heart. The standard surgical repair or replacement procedure is a highly invasive operation with significant concomitant risks including bleeding, infection, stroke, heart attack, arrhythmia, renal failure, adverse reactions to the anesthesia medications, sudden death. The complications are significant enough to exclude many patients from surgical treatment.

Patent publication US 2011/0137397 A1 discloses a prosthetic heart valve for implantation within a mitral valve of a patient, including a radially compressible main body and a one-way valve portion. A ventricular anchor, coupled to the main body and disposed outside of the main body, is provided in the prosthetic heart valve.

In conclusion, for replacing a patient's heart valve endovascularly or transcatheterly, there is a great need to provide a novel medical device and method that can avoid the above discussed problems.

### SUMMARY OF INVENTION

The present invention aims to provide physicians a device which can avoid a traumatic surgical procedure and instead provide a medical device that can be implanted through a catheter-based, less invasive procedure for valve replacement treatment.

This invention generally relates to a medical device for replacing a patient's heart valve endovascularly or transcatheterly. The device includes: a delivery system having an entry profile of 30 French (Fr) or less; a self-expandable annulus ring disposed in the delivery system; and a replacement heart valve disposed in the delivery system. The self-expandable annulus ring includes a self-expandable anchoring and clipping structure and multiple bridges connected to the anchoring and clipping structure. The replacement heart valve is connected to the bridges. The bridges are formed of a shape memory material; during deployment, the bridges fold upwardly inside the self-expandable anchoring and clipping structure to lift the replacement heart valve into position.

The invention, as defined by claim 1, provides a low profile heart valve system for endovascularly or transcatheterly replacing a patient's heart valve, which includes: a delivery system including a tube; a self-expandable annulus ring disposed in the tube of the delivery system, the self-expandable annulus ring including: a self-expandable anchoring and clipping structure; and a plurality of bridges, each having a first end and a second end, the first end being connected to the anchoring and clipping structure; and a replacement heart valve disposed in the tube of the delivery system and connected to the second ends of the plurality of bridges; wherein the second end of each bridge is located below the first end when the bridges are disposed in the tube of the delivery system, wherein each bridge is formed of a shape memory material with a memorized shape, wherein the memorized shape of the bridge is one in which the bridge folds inwardly and upwardly from its first end and in which the second end of the bridge is located above the first end.

In some embodiments, the anchoring and clipping structure includes: an annulus top flange; an annulus supporting body connected at its upper end to the annulus top flange; an anchoring feature having a first end connected to a lower end of the annulus supporting body; a plurality of annulus top flange locking hoops joined to an upper end of the annulus top flange; and a plurality of anchoring feature locking hoops joined to a second end of the anchoring feature; wherein the first end of each bridge is connected to the lower end of the annulus supporting body.

In some embodiments, the second end of the anchoring feature is located below the first end when the anchoring feature is disposed in the tube of the delivery system, the anchoring feature is formed of a shape memory material with a memorized shape, and the memorized shape of the anchoring feature is one in which the anchoring feature folds upwardly from its first end and then downwardly outside of the annulus supporting body.

In some embodiments, the replacement heart valve includes: a leaflet supporting body having an upper end connected to second ends of the plurality of bridges; and a plurality of leaflets mounted on the leaflet supporting body.

In another aspect, not according to the invention, this disclosure provides a method for endovascularly or transcatheterly placing a heart valve in a patient's heart, which includes: providing a heart valve system, the heart valve system including a delivery system having a tube, and a self-expandable annulus ring and a replacement heart valve disposed in the tube, wherein the self-expandable annulus ring includes a self-expandable anchoring and clipping structure and a plurality of bridges, each bridge having a first end and a second end, the first end being connected to the anchoring and clipping structure, wherein the second end of each bridge is located below the first end when the bridges are disposed in the tube of the delivery system, wherein each bridge is formed of a shape memory material having a memorized shape, and wherein the memorized shape of each bridge is one in which the bridge folds inwardly and upwardly from its first end and in which the second end of the bridge is located above the first end; inserting the heart valve system to a location near a native valve of a patient's heart; releasing the self-expandable annulus ring including the bridges from the tube, without releasing the replacement heart valve; and then releasing the replacement heart valve from the tube.

In some embodiment, the anchoring and clipping structure includes: an annulus top flange; an annulus supporting body connected at its upper end to the annulus top flange; an anchoring feature having a first end connected to a lower end of the annulus supporting body; a plurality of annulus top flange locking hoops joined to an upper end of the annulus top flange; and a plurality of anchoring feature locking hoops joined to a second end of the anchoring feature; wherein the first end of each bridge is connected to the lower end of the annulus supporting body.

In some embodiment, in the anchoring and clipping structure, the second end of the anchoring feature is located below the first end when the anchoring feature is disposed in the tube of the delivery system, the anchoring feature is formed of a shape memory material with a memorized shape, and the memorized shape of the anchoring feature is one in which the anchoring feature folds upwardly from its first end and then downwardly outside of the annulus supporting body.

In some embodiment, the replacement heart valve includes: a leaflet supporting body having an upper end connected to second ends of the plurality of bridges; and a plurality of leaflets mounted on the leaflet supporting body.

### DESCRIPTION OF THE FIGURES

The aforementioned and other features of this invention and the manner of obtaining them will become more apparent, and will be best understood by reference to the following description, taken in conjunction with the accompanying drawings. These drawings depict only a typical embodiment of the invention and do not therefore limit its scope. They serve to add specificity and detail.
Figure 1 shows an exemplary configuration of the device and its functional components according to an embodiment of the present invention.
Figure 2 shows a side view of the device fully self-expanded.
Figure 3 shows another view of a part of the device and its functional components.
Figure 4, Figure 5 and Figure 6 are cross-sectional views showing relative positions of the device's functional components and native heart valve structures during deployment.
Figure 7 shows a top view of the device after the first step of deployment.
Figure 8 shows a side view of the device after the first step of deployment.
Figure 9 is a cross-sectional view showing the relative positions of the device's functional components and the native heart valve structures after the second step of deployment.
Figure 10 shows a top view of the device after the second step of deployment.
Figure 11 shows a side view of the device after the second step of deployment.

### DETAILED DESCRIPTION OF THE PREFERRED EMBODIMENTS

This invention generally relates to medical devices for endovascularly of transcatheterly replacing a patient's heart valve. None of the methods herein described form part of the invention, which is defined solely by the claims.

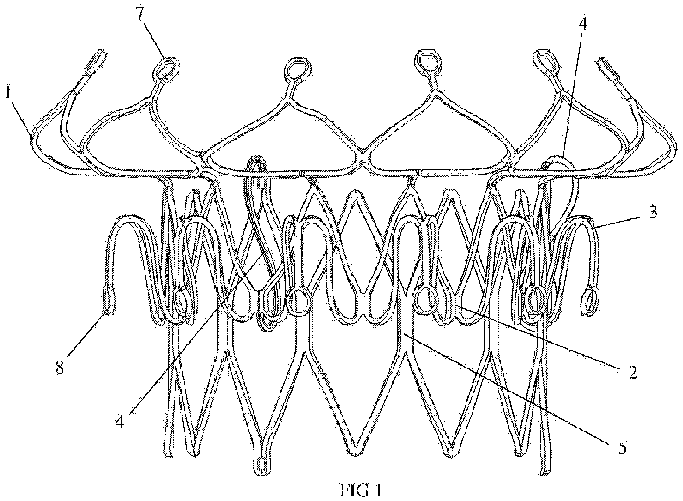

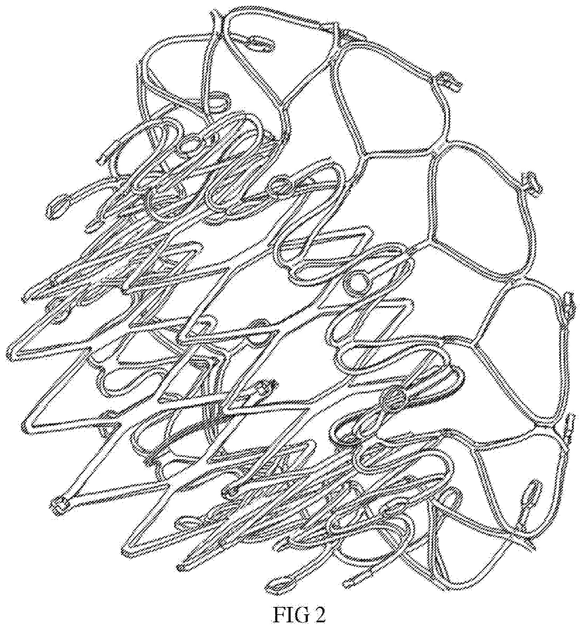

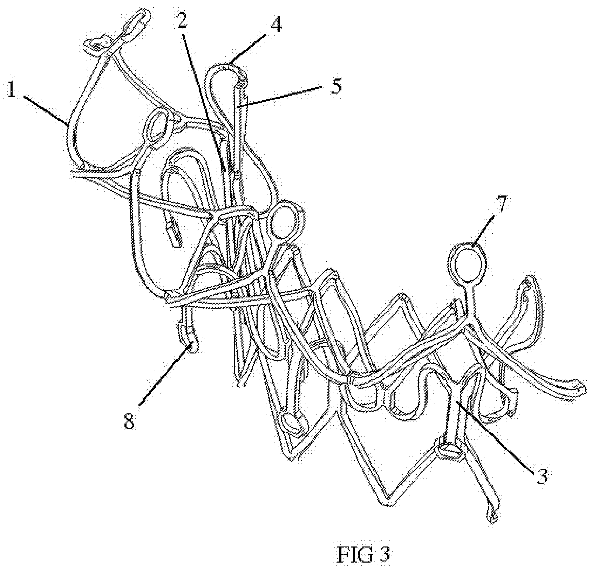

One aspect of the present invention provides a medical device that comprises three parts: a delivery system (9), a self-expandable annulus ring (10), and a replacement heart valve (11) which is connected to the self-expandable annulus ring (10). As shown in Figs. 1-11, the self-expandable annulus ring (10) includes an annulus top flange (also referred to as an annulus top ring) (1), an annulus supporting body (2), an anchoring feature (3), bridges (4), annulus top flange locking hoops (7) and anchoring feature locking hoops (8). The replacement heart valve (11) includes a leaflet supporting body (5) and leaflets (6). The self-expandable annulus ring (10) acts as the positioning and locking functional portion of the heart valve.

The self-expandable annulus ring (10) and the leaflet supporting body (5) are formed of wire or tubing materials; in some embodiments, they may be fabricated from a single piece of super elastic or shape memory tubing. The leaflets (6) may be made from polymers or animal tissues.

The delivery system (9) includes a tube. Before deployment, the self-expandable annulus ring (10) and the replacement heart valve (11) are in an un-expanded state and stored inside the tube of the delivery system (9).

The shape of the device in the fully self-expanded state is described with reference to Figs. 1-3 and 9-11. In the descriptions below, the atrial end of the device is referred to as the "top" and the ventricular end the "bottom", and terms such as up, down, above, below, etc. are used in this sense. Also, "outside" means relatively farther away form the rotational axis of the device, and "inside" means relatively closer to the rotational axis. It should be appreciated that these terms are relative and used only for convenience of the description.

In the expanded state, the annulus top flange (1), the annulus supporting body (2), the anchoring feature (3), the and leaflet supporting body (5) all have generally annular shapes around a center opening of the device. The annulus top flange (1) is located at the top of the device. It extends radially outwardly in a direction generally perpendicular to the rotational axis, and then bends upwardly and inwardly. The annulus top flange locking hoops (7) are provided at the top of the annulus top flange (1) and are used to control the release of the annulus top flange (1) from the delivery system (9).

The annulus supporting body (2) is located below and connected to the inner end of the annulus top flange (1), and extends in the axial direction. The anchoring feature (3) is connected to the bottom of the annulus supporting body (2). From the end where it is connected to the annulus supporting body (2), the anchoring feature (3) folds upwards and then downwards, outside of the annulus supporting body (2). The anchoring feature locking hoops (8) are provided at the other end of the anchoring feature (3) (i.e. the end not connected to the annulus supporting body (2)) and are used control the release of the anchoring feature (3) from the delivery system (9).

The bridges (4) are connected at their first ends to the bottom of the annulus supporting body (2), and fold upwardly inside of the annulus supporting body (2). The leaflet supporting body (5) is connected at its top to the second end of the bridges (4) (i.e. the end of the bridges (4) that is not connected to the annulus supporting body (2)). The leaflet supporting body (5) is located inside the annulus supporting body (2) and extends in the axial direction, and the leaflets (6) are mounted on the leaflet supporting body (5) in the center opening of the device.

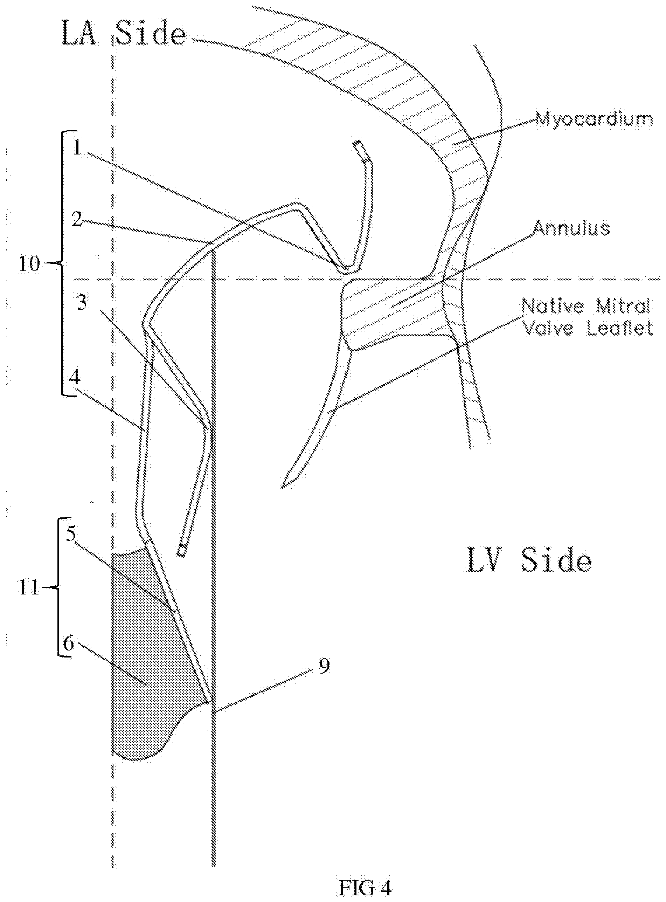

In the un-expended state as stored inside the delivery system (9), the anchoring feature (3) and the bridges (4) are not folded upwardly from the ends where they are connected to the bottom of the annulus supporting body (2). Rather, they are disposed below the annulus supporting body (2), and the leaflet supporting body (5) is disposed below the bridges (4), as seen in Fig. 4.

The deployment (self-expansion) of the self-expandable annulus ring (10) and replacement heart valve (11) is described with reference to Figs. 4-11. Figs. 4-6 and 9 are schematic cross-sectional views of the device along with relevant anatomical features of the heart. The vertical dashed lines in Figs. 4-6 and 9 indicate the rotational axis of the device, and only one half of the device to the right of the rotational axis is shown in these views.

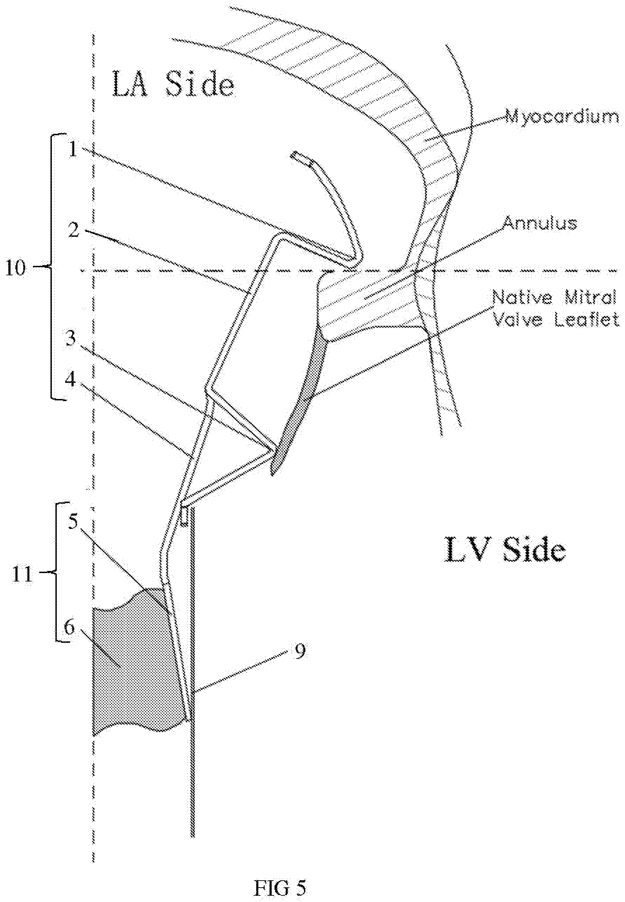

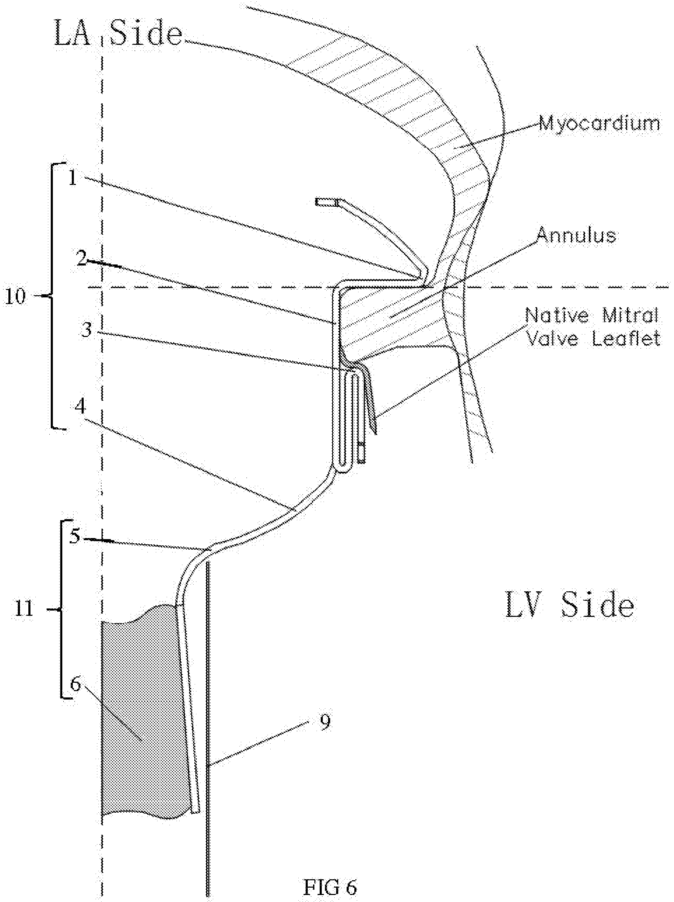

During the deployment of the device, the first step is to release the self-expandable annulus ring (10) from the delivery system (9), as shown in Figs. 4-6. In Fig. 4, the annulus top flange (1) and an upper portion of the annulus supporting body (2) have been released from the delivery system (9). In Fig. 5, the anchoring feature (3) except for the anchoring feature locking hoops (8), and a portion of the bridges (4), have also been released. In Fig. 6, the anchoring feature locking hoops (8) and most of bridges (4) have also been released.

As shown in Figs. 4-6, the horizontally radially extending flange of the annulus top flange (1) is configured to be seated on the top of the native heart valve annulus when fully expanded. The anchoring feature (3) is useful to engage or capture the native heart valve leaflet, as shown in Fig. 6 where the anchoring feature (3) has been fully released and has folded upwards from where it is connected to the annulus supporting body (2). After releasing, the annulus top flange (1), the anchoring feature (3), and the annulus supporting body (2) will be self-expanded to the diameter of the heart valve annulus (see Fig. 6), to support the heart valve annulus and the whole device. The annulus top flange (1), the annulus supporting body (2), and the anchoring feature (3) collectively forms an anchoring and clipping structure; when fully expanded (see, e.g., Fig. 6), the clipping structure is configured to function as "clips", to place and secure the whole device in the right heart valve annulus position.

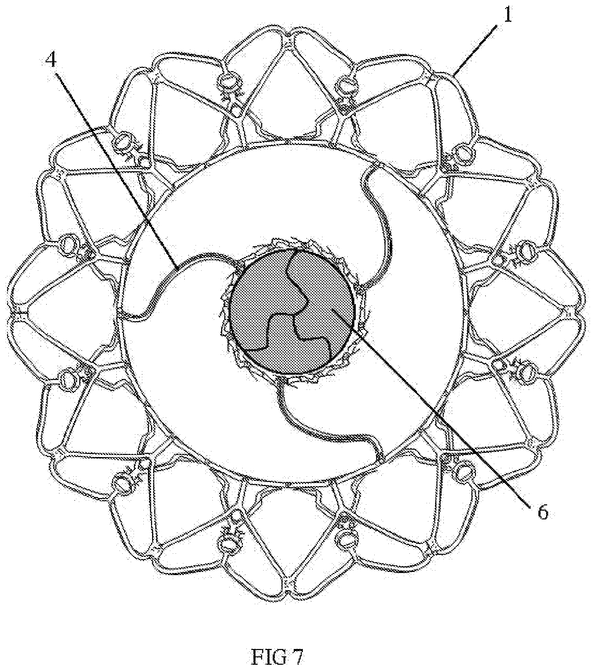

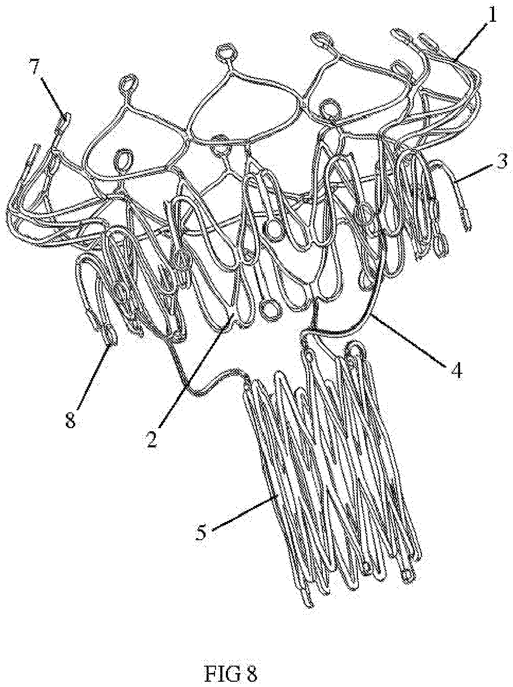

Figs. 7-8 also illustrate the device in the same state as shown in Fig. 6, i.e., after the first step of deployment. Fig. 7 is a top view, and Fig. 8 is a side perspective view.

The second step of the deployment is to release the replacement heart valve (11) from the delivery system (9).

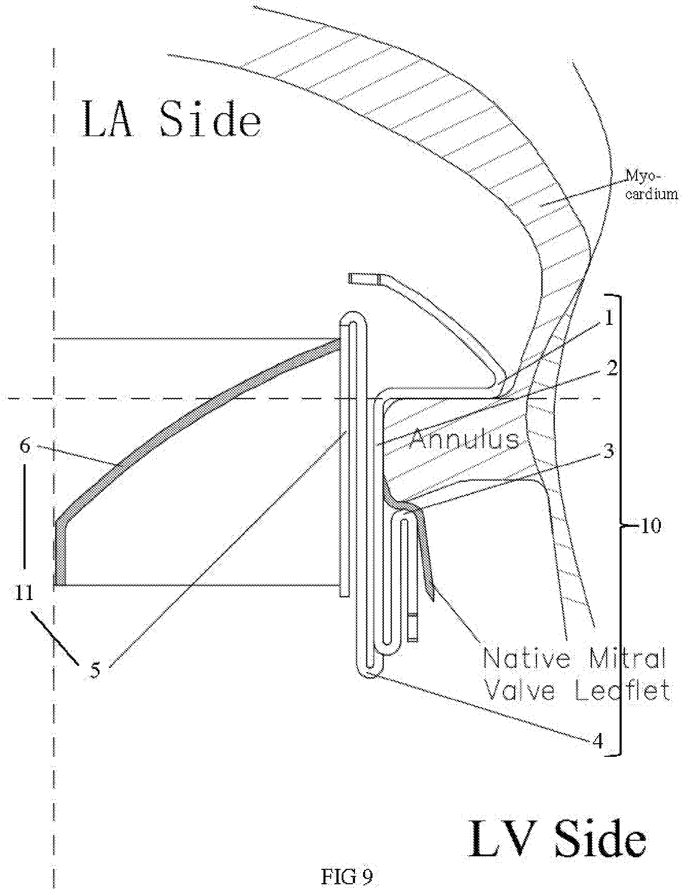

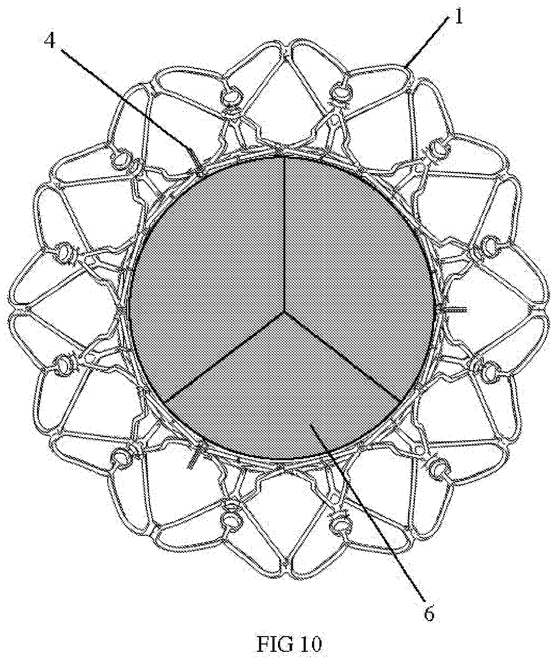

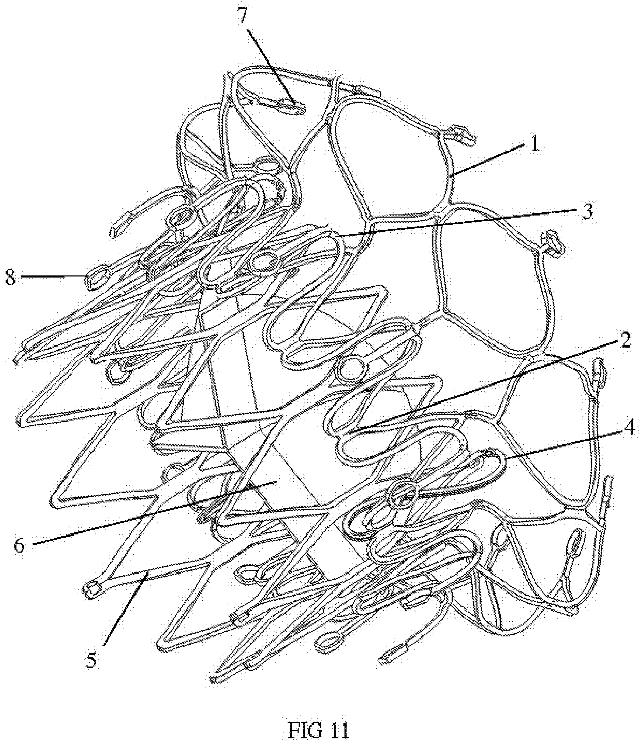

After releasing the bridges (4) and the leaflet supporting body (5), the bridges (4), which are fabricated from super elastic or shape memory material, will go back to its memorized shape, by folding inwardly and upwardly from its first end which is connected to the bottom of the annulus supporting body (2), as shown in Fig. 9. This causes the bridges (4) to lift themselves and the leaflet supporting body (5) (which is connected to the second end of the bridges (4)) up, engage themselves with the annulus supporting body (2) and place the leaflet supporting body (5) inside in the center of the annulus supporting body (2). As the bridges (4) are completely folded upwards, the leaflet supporting body (5) is in the expanded state, as shown in Fig. 9. In other words, the leaflet supporting body (5) and the leaflets (6) can be positioned automatically with the bridges (4) self-expansion. Figs. 10-11 also illustrate the device in the same state as shown in Fig. 9, i.e., after the second step of deployment. Fig. 10 is a top view, and Fig. 11 is a perspective view.

By separately deploying the self-expandable annulus ring (10) and the heart valve body (5) with leaflets (6), the entry profile of the device can be 30 Fr or less (including the delivery system).

The above examples are for illustrative purposes only and are in no way meant to limit the invention. They are given to aid in understanding the invention, but it is to be understood that the invention is not limited to the particular materials or procedures of examples. The Examples are provided by way of illustration only and not by way of limitation. The parameters and data are not to be construed to limit the scope of the embodiments of the invention.

## Claims

1. A low profile heart valve system for endovascularly or transcatheterly replacing a patient's heart valve, comprising:
a delivery system (9) including a tube;
a self-expandable annulus ring (10) disposed in the tube of the delivery system (9), the self-expandable annulus ring (10) including:
a self-expandable anchoring and clipping structure; and
a plurality of bridges (4), each having a first end and a second end, the first end being connected to the anchoring and clipping structure; and
a replacement heart valve (11) disposed in the tube of the delivery system (9) and connected to the second ends of the plurality of bridges (4);
wherein the second end of each bridge (4) is located below the first end when the bridges (4) are disposed in the tube of the delivery system (9), wherein each bridge (4) is formed of a shape memory material with a memorized shape, wherein the memorized shape of the bridge (4) is one in which the bridge (4) folds inwardly and upwardly from its first end and in which the second end of the bridge (4) is located above the first end.

2. The low profile heart valve system of claim 1, wherein the anchoring and clipping structure includes:
an annulus top flange (1);
an annulus supporting body (2) connected at its upper end to the annulus top flange (1);
an anchoring feature (3) having a first end connected to a lower end of the annulus supporting body (2);
a plurality of annulus top flange locking hoops (7) joined to an upper end of the annulus top flange (1); and
a plurality of anchoring feature locking hoops (8) joined to a second end of the anchoring feature (3);
wherein the first end of each bridge (4) is connected to the lower end of the annulus supporting body (2).

3. The low profile heart valve system of claim 2, wherein the second end of the anchoring feature (3) is located below the first end when the anchoring feature (3) is disposed in the tube of the delivery system (9), wherein the anchoring feature (3) is formed of a shape memory material with a memorized shape, and wherein the memorized shape of the anchoring feature (3) is one in which the anchoring feature (3) folds upwardly from its first end and then downwardly outside of the annulus supporting body (2).

4. The low profile heart valve system of claim 2, wherein the replacement heart valve includes:
a leaflet supporting body (5) having an upper end connected to second ends of the plurality of bridges (4); and
a plurality of leaflets (6) mounted on the leaflet supporting body (5).

## Patentansprüche

1. Herzklappensystem mit niedrigem Profil zum endovaskulären oder transkatheterischen Ersetzen einer Herzklappe eines Patienten, das Folgendes umfasst:
ein Zuführungssystem (9), das einen Schlauch umfasst;
einen selbstexpandierbaren Kreisring (10), der in dem Schlauch des Zuführungssystems (9) angeordnet ist, wobei der selbstexpandierbare Kreisring (10) Folgendes umfasst:
eine selbstexpandierbare Verankerungs- und Klammerstruktur; und
eine Vielzahl von Brücken (4), die jeweils ein erstes Ende und ein zweites Ende aufweisen, wobei das erste Ende mit der Verankerungs- und Klammerstruktur verbunden ist; und
eine Ersatzherzklappe (11), die in dem Schlauch des Zuführungssystems (9) angeordnet ist und mit den zweiten Enden der Vielzahl von Brücken (4) verbunden ist;
wobei das zweite Ende von jeder Brücke (4) sich unter dem ersten Ende befindet, wenn die Brücken (4) in dem Schlauch des Zuführungssystems (9) angeordnet sind, wobei jede Brücke (4) aus einem Formgedächtnismaterial mit einer ins Gedächtnis eingeprägten Form gebildet ist, wobei die ins Gedächtnis eingeprägte Form der Brücke (4) eine ist, in der die Brücke (4) sich von ihrem ersten Ende nach innen und nach oben faltet und in der das zweite Ende der Brücke (4) sich über dem ersten Ende befindet.

2. Herzklappensystem mit niedrigem Profil nach Anspruch 1, wobei die Verankerungs- und Klammerstruktur Folgendes umfasst:
einen oberen Kreisflansch (1);
einen Kreisstützkörper (2), der an seinem oberen Ende mit dem oberen Kreisflansch (1) verbunden ist;
ein Verankerungsmerkmal (3), das ein erstes Ende aufweist, das mit einem unteren Ende des Kreisstützkörpers (2) verbunden ist;
eine Vielzahl von oberen Kreisflansch-Verriegelungsreifen (7), die mit einem oberen Ende des oberen Kreisflansches (1) verbunden sind; und
eine Vielzahl von Verankerungsmerkmal-Verriegelungsreifen (8), die mit einem zweiten Ende des Verankerungsmerkmals (3) verbunden sind;
wobei das erste Ende von jeder Brücke (4) mit dem unteren Ende des Kreisstützkörpers (2) verbunden ist.

3. Herzklappensystem mit niedrigem Profil nach Anspruch 2, wobei das zweite Ende des Verankerungsmerkmals (3) sich unter dem ersten Ende befindet, wenn das Verankerungsmerkmal (3) in dem Schlauch des Zuführungssystems (9) angeordnet ist, wobei das Verankerungsmerkmal (3) aus einem Formgedächtnismaterial mit einer ins Gedächtnis eingeprägten Form gebildet ist, und wobei die ins Gedächtnis eingeprägte Form des Verankerungsmerkmals (3) eine ist, in welcher das Verankerungsmerkmal (3) sich von seinem ersten Ende nach oben und dann außerhalb des Kreisstützkörpers (2) nach unten faltet.

4. Herzklappensystem mit niedrigem Profil nach Anspruch 2, wobei die Ersatzherzklappe Folgendes umfasst:
einen Blattstützkörper (5), der ein oberes Ende aufweist, das mit zweiten Enden der Vielzahl von Brücken (4) verbunden ist; und
eine Vielzahl von Blättern (6), die an dem Blattstützkörper (5) montiert sind.

## Revendications

1. Système de valve cardiaque à profil bas pour le remplacement endovasculaire ou transcathéter d'une valve cardiaque d'un patient, comprenant
un système d'alimentation (9) comprenant un tuyau;
un anneau auto-expansible (10) disposé dans le tuyau du système d'alimentation (9), l'anneau auto-expansible (10) comprenant :
une structure d'ancrage et d'agrafage auto-expansible ; et
une pluralité de ponts (4), chacun ayant une première extrémité et une seconde extrémité, la première extrémité étant reliée à la structure d'ancrage et d'agrafage ; et
une valve cardiaque de remplacement (11) disposée dans le tube du système d'alimentation (9) et reliée aux secondes extrémités de la pluralité de ponts (4) ;
dans lequel la seconde extrémité de chaque pont (4) est située en dessous de la première extrémité lorsque les ponts (4) sont disposés dans le tube du système d'alimentation (9), chaque pont (4) étant formé d'un matériau à mémoire de forme ayant une forme mémorisée, la forme mémorisée du pont (4) étant celle dans laquelle le pont (4) se plie vers l'intérieur et vers le haut à partir de sa première extrémité et dans laquelle la seconde extrémité du pont (4) est située au-dessus de la première extrémité.

2. Système de valves cardiaque à profil bas selon la revendication 1, dans lequel la structure d'ancrage et d'agrafage comprend les éléments suivants:
une bride annulaire supérieure (1);
un corps de support annulaire (2) qui est relié à son extrémité supérieure à la bride annulaire supérieure (1) ;
un élément d'ancrage (3) ayant une première extrémité reliée à une extrémité inférieure du corps de support annulaire (2);
une pluralité d'arceaux de verrouillage de la bride annulaire supérieure (7) reliés à une extrémité supérieure de la bride annulaire supérieure (1); et
une pluralité d'arceaux de verrouillage d'élément d'ancrage (8) reliés à une seconde extrémité de l'élément d'ancrage (3) ;
la première extrémité de chaque pont (4) étant reliée à l'extrémité inférieure du corps de support annulaire (2).

3. Système de valve cardiaque à profil bas selon la revendication 2, dans lequel la deuxième extrémité de l'élément d'ancrage (3) est située sous la première extrémité lorsque l'élément d'ancrage (3) est placée dans le tube du système d'alimentation (9), dans lequel l'élément d'ancrage (3) est formé d'un matériau à mémoire de forme ayant une forme mémorisée, et dans lequel la forme mémorisée de l'élément d'ancrage (3) est celle dans laquelle l'élément d'ancrage (3) se plie vers le haut à partir de sa première extrémité et ensuite vers le bas à l'extérieur du corps de support annulaire (2).

4. système de valve cardiaque à profil bas selon la revendication 2, dans lequel la valve cardiaque de remplacement comprend les éléments suivants :
un corps de support de feuille (5) ayant une extrémité supérieure reliée à des secondes extrémités de la pluralité de ponts (4) ; et
une pluralité de pales (6) montées sur le corps de support de pales (5).
